(19) Europäisches Patentamt
European Patent Office
Office européen des brevets

(11) Publication number: 0 123 473
A2

# EUROPEAN PATENT APPLICATION

(21) Application number: 84302414.2

(22) Date of filing: 09.04.84

(51) Int. Cl.³: C 07 D 417/14
C 07 D 413/14, C 07 D 519/00
C 07 D 473/30, C 07 D 473/34
A 61 K 31/535
//(C07D519/00, 473/00)

(30) Priority: 25.04.83 GB 8311228

(43) Date of publication of application: 31.10.84 Bulletin 84/44

(84) Designated Contracting States: AT BE CH DE FR GB IT LI LU NL SE

(71) Applicant: Fujisawa Pharmaceutical Co., Ltd.
3, Doshomachi 4-chome Higashi-ku
Osaka-shi, Osaka 541(JP)

(72) Inventor: Takaya, Takao
1-5-87 Suimeidai Kawanishi-shi
Hyogo 666-01(JP)

(72) Inventor: Tozuka, Zenzaburo
No. 4-8-A-1107, Kamishinden
Toyonaka-shi Osaka 565(JP)

(74) Representative: Shipley, Warwick Grenville Michael
VENNER, SHIPLEY & CO. 368 City Road
London EC1V 2QA(GB)

(54) **Morpholine derivatives, processes for the preparation thereof, the use thereof and pharmaceutical compositions containing them.**

(57) The present invention provides compounds of the formula:

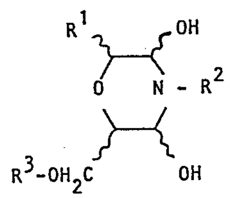

wherein $R^1$ is a uracilyl, cytosinyl, hypoxanthinyl, adeninyl or thiazolyl radical, which may be substituted by halogen or carbamoyl;

$R^2$ is a nitrogen-containing unsaturated heterocyclic radical, which may be substituted by oxo or lower alkyl; and

$R^3$ is a hydrogen atom or a phosphono group;

and the pharmaceutically-acceptable salts thereof.

The present invention also provides processes for the preparation of these compounds and pharmaceutical compositions containing them and provides a method of treating cancer which comprises administering these compounds to humans and animals.

EP 0 123 473 A2

Morpholine derivatives, processes for the preparation thereof, the use thereof and pharmaceutical compositions containing them

The present invention relates to new morpholine derivatives. More particularly, the present invention relates to new morpholine derivatives and to the pharmaceutically-acceptable salts thereof, which have anti-tumour activity, to processes for the preparation thereof, to pharmaceutical compositions comprising them and to a method of use thereof.

Accordingly, one object of the present invention is to provide new and useful morpholine derivatives and pharmaceutically-acceptable salts thereof.

Another object of the present invention is to provide processes for preparation of these morpholine derivatives and of the pharmaceutically-acceptable salts thereof.

A further object of the present invention is to provide a pharmaceutical composition comprising the new morpholine derivative or pharmaceutically-acceptable salt thereof as an anti-tumour agent.

Yet a further object of the present invention is to provide a method of using the new morpholine

derivatives and the pharmaceutically-acceptable salts thereof for the therapeutic treatment of cancer in human beings and animals.

The new morpholine derivatives of the present invention are compounds of the following general formula (I):

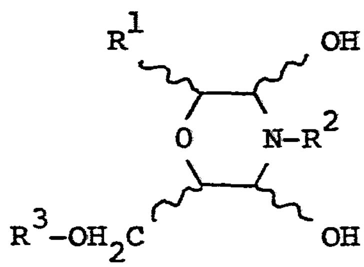

(I)

wherein $R^1$ is a uracilyl, cytosinyl, hypoxanthinyl, adeninyl or thiazolyl radical, which may be substituted by halogen or carbamoyl,

$R^2$ is a nitrogen-containing unsaturated heterocyclic radical, which may be substituted by oxo or lower alkyl, and

$R^3$ is a hydrogen atom or a phosphono group,

and the pharmaceutically-acceptable salts thereof.

Examples and illustrations of the above definitions are explained in detail as follows.

The term "lower" is intended to mean 1 to 6 carbon atoms, unless otherwise indicated.

The heterocyclic group $R^1$ is uracilyl, cytosinyl, hypoxanthinyl, adeninyl or thiazolyl, which may be substituted by halogen, for example fluorine, chlorine, bromine or iodine, or by carbamoyl.

Examples of the heterocyclic group having such substituent(s) include halogenated heterocyclic radicals, such as 5-fluorouracilyl, 5-chlorouracilyl, 5-bromouracilyl, 6-fluorouracilyl, 6-chlorouracilyl, 5-fluorocytosinyl, 5-chlorocytosinyl, 6-fluorocytosinyl, 2-fluorohypoxanthinyl, 8-chlorohypoxanthinyl, 8-chloro-

adeninyl and 2-chlorothiazolyl; and carbamoyl-substituted heterocyclic radicals, such as 2-carbamoylthiazolyl, 4-carbamoylthiazolyl or the like.

The nitrogen-containing unsaturated heterocyclic radical $R^2$ is an unsaturated mono- or polycyclic radical containing at least one nitrogen atom.

Examples of the heterocyclic radical $R^2$ may be unsaturated 3- to 9-membered and preferably 5- or 6-membered, monocyclic heterocyclic radicals containing 1 to 4 nitrogen atoms, such as pyrrolyl, imidazolyl, pyrazolyl, triazolyl, [e.g. 1H-1,2,3-triazolyl, 1H-1,2,4-triazolyl, 2H-1,2,3-triazolyl, 4H-1,2,4-triazolyl, etc.], tetrazolyl [e.g. 1H-tetrazolyl, 2H-tetrazolyl, etc.], pyridyl, pyrimidinyl, pyrazinyl, pyridazinyl or the like; unsaturated fused heterocyclic radicals containing 1 to 4 nitrogen atoms, such as indolyl, benzimidazolyl, benzopyrazolyl, benzotriazolyl, purinyl or the like; unsaturated 3- to 9-membered and preferably 5- or 6-membered, monocyclic heterocyclic radicals containing 1 to 4 nitrogen atoms and 1 or 2 sulphur or oxygen atoms, such as thiazolyl, isothiazolyl, thiadiazolyl [e.g. 1,2,3-thiadiazolyl, 1,2,4-thiadiazolyl, 1,3,4-thiadiazolyl, 1,2,5-thiadiazolyl, etc.], thiazolinyl, oxazolyl, isoxazolyl, oxadiazolyl [e.g. 1,2,4-oxadiazolyl, 1,3,4-oxadiazolyl, 1,2,5-oxadiazolyl, etc.] or the like.

The above-mentioned heterocyclic radicals $R^2$ may be substituted by oxo or lower alkyl [e.g. methyl, ethyl, propyl, isopropyl, butyl, isobutyl, sec.-butyl, tert.-butyl, pentyl, hexyl, etc.]. Examples of heterocyclic radicals containing such substituent(s) include 2-methylthiazolyl, 5-methyloxazolyl, hypoxanthinyl and the like.

The pharmaceutically-acceptable salts of the new compounds (I) include conventional non-toxic salts, for example metal salts, such as the alkali metal salts [e.g. sodium salts, potassium salts, etc.], the alkaline earth metal salts [e.g. calcium salts, magnesium salts, etc.], the ammonium salts, the salts with organic bases [e.g. trimethylamine salts, triethylamine salts, pyridine salts, picoline salts, dicyclohexylamine salts, N,N'-dibenzylethylenediamine salts, etc.], the salts with organic acids [e.g. formates, acetates, trifluoroacetates, maleates, tartrates, methanesulphonates, benzenesulphonates, toluenesulphonates, etc.], the salts with inorganic acids [e.g. hydrochlorides, hydrobromides, sulphates, phosphates, etc.], and the salts with amino acids [e.g. arginine salts, ornithine salts, etc.] and the like.

The new compounds (I) and the salts thereof according to the present invention can be prepared by the following processes:

Process 1.

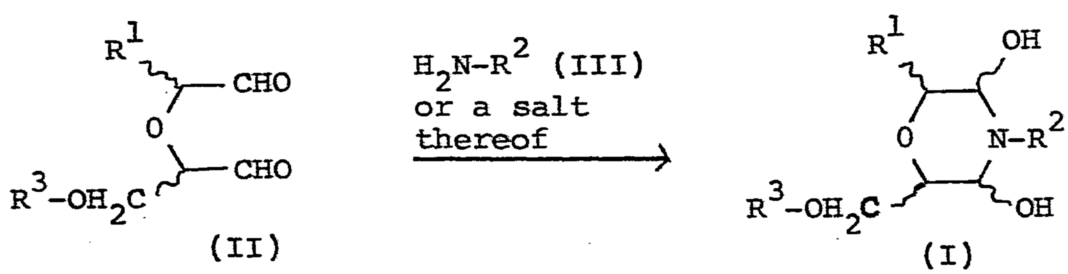


(II) or a salt thereof → (I) or a salt thereof

Process 2.

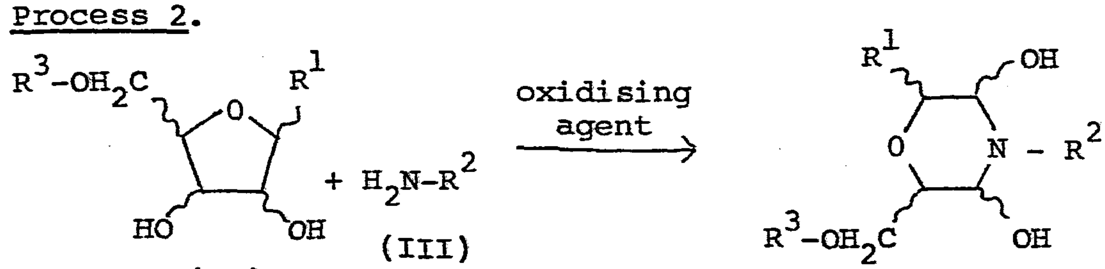


(IV) or a salt thereof + (III) or a salt thereof → (I) or a salt thereof

wherein $R^1$, $R^2$ and $R^3$ are as defined above.

The processes for preparing the new compounds (I) and the salts thereof are explained in detail in the following:

Process 1

The new compounds (I) and the salts thereof can be prepared by reacting a compound (II) or a salt thereof with a compound (III) or a salt thereof.

Examples of salts of the compounds (II) and (III) include those exemplified above for the compounds (I).

This reaction is usually carried out in a conventional solvent, such as water, an alcohol [e.g. methanol, ethanol, propanol, etc.], tetrahydrofuran, dioxan, dimethylformamide, dimethyl sulphoxide or any other organic solvent which does not adversely influence the reaction, or a mixture thereof.

The reaction is preferably carried out in the presence of an acid, such as inorganic acid [e.g. hydrochloric acid, hydrobromic acid, sulphuric acid, phosphoric acid, polyphosphoric acid, etc.] or an organic acid [e.g. acetic acid, trifluoroacetic acid, benzenesulphonic acid, toluenesulphonic acid, etc.] or the like. If the acid used is a liquid, it can also be used as a solvent.

The reaction temperature is not critical, the reaction usually being carried out at ambient temperature or with warming.

Process 2

The new compounds (I) and the salts thereof can be prepared by reacting a compound (IV) or a salt thereof with a compound (III) or a salt thereof in the presence of an oxidising agent.

Examples of salts of the compounds (IV) and (III)

include those exemplified above for the compounds (I).

The oxidising agents used for this reaction include those conventionally used for the oxidation of the 1,2-diol function, such as periodic acid or its salts [e.g. metaperiodic acid, sodium metaperiodate, potassium metaperiodate, paraperiodic acid, sodium paraperiodate, potassium paraperiodate, etc.], lead compounds [e.g. lead tetraacetate, lead tetrabenzoate, etc.], chromic acid or its salts [e.g. sodium dichromate, potassium dichromate, etc.] or the like.

This reaction is usually carried out in a conventional solvent, such as water, an alcohol [e.g. methanol, ethanol, t-butyl alcohol, etc.], dioxan, acetic acid or any other organic solvent which does not adversely influence the reaction, or a mixture thereof.

The reaction temperature is not critical, the reaction usually being carried out with cooling or with warming.

The starting compounds (II) include known compounds, such as are described in CARBOHYDRATE RESEARCH, 54(1), 75-84/1977, CANCER RESEARCH, 40(3), 598-603/1980, etc., and new compounds. The new starting compounds can be prepared by the following method:

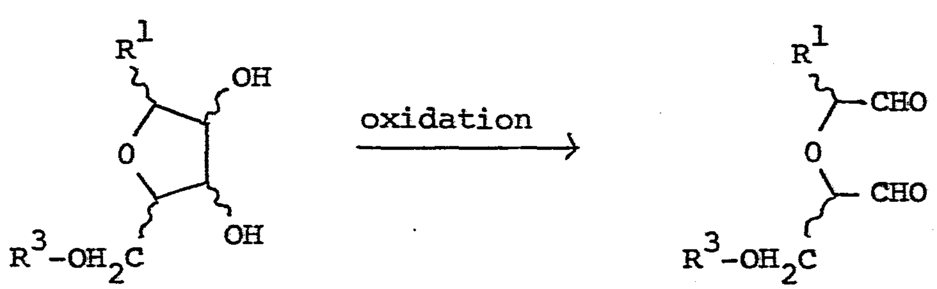


(IV) or a salt thereof

(II) or a salt thereof

wherein $R^1$ and $R^3$ are as defined above.

Compounds (II) and the salts thereof can be prepared by oxidising compounds (IV) or the salts thereof.

The oxidising agents used for this reaction can be the same as those exemplified in the explanation of Process 2.

This reaction is usually carried out in a conventional solvent, such as water, an alcohol [e.g. methanol, ethanol, t-butyl alcohol, etc.[, dioxan, acetic acid, or a mixture thereof, the solvents being selected according to the nature of the oxidising agent used. The reaction temperature is not critical, the reaction usually being carried out with cooling or with warming.

The compounds (II) can be isolated in a conventional manner, such as by using an ion-exchange resin column, lyophilisation or the like, but it can optionally be used in the next step, i.e. Process 1, without isolation.

It is to be noted that new compounds (I) and the starting compounds (II), (III) and (IV) include one or more stereoisomers, due to asymmetric carbon atoms in the molecule, and tautomers. All such isomers of the compounds (I), (II), (III) and (IV) are included within the scope of the present invention.

The new morpholine derivatives (I) and the pharmaceutically-acceptable salts thereof possess an anti-tumour activity and are useful for the therapeutic treatment of cancer.

For the purpose of showing the pharmacological activity of the new compounds (I), test data on anti-tumour activity are illustrated in the following:

Test Method

Six male B D $F_1$ mice, aged more than 6 weeks and weighing 22.6 g. to 25.9 g., were used per group. Lymphocytic Leukemia P388 was transferred every 6 or 7 days in DBA/2 mice by intraperitoneal inoculation of ascites cells. The test compounds were dissolved in phosphate buffer serum (PBS). After 24 hours of the inoculation of Leukemia cells to the test mice, the test compound was administered intraperitoneally in doses of 32, 56, 100, 320 mg/kg, respectively, to each treatment group (PBS only in the control group), once a day for 4 days. The anti-tumour activity of the test compound was evaluated by the increase in life span in comparison with the control (ILS = T/C x 100-100) in leukemias, wherein T is the medium survival time (MST) of the treated group and C is the medium survival time of the control group.

Test result

The test results obtained are shown in the following Table 1.

Table 1

| Test Compound (Example No.) | Dose (mg/kg) | ILS ( % ) |
|---|---|---|
| Example 1 | 32 | 55 |
| | 100 | 100 |
| Example 2 | 100 | 75 |
| Example 3 | 32 | 30 |
| | 100 | 85 |
| | 320 | 300 |
| Example 4 | 32 | 30 |
| | 100 | 60 |
| | 320 | 90 |
| Example 6 | 32 | 30 |
| | 100 | 80 |
| Example 7 | 32 | 60 |
| | 100 | 80 |
| Example 8 | 32 | 45 |
| | 100 | 70 |
| Example 12 | 32 | 46 |
| | 100 | 67 |
| Example 14 | 32 | 60 |
| | 100 | 85 |
| | 320 | 295 |
| Example 16 | 32 | 55 |
| | 100 | 80 |

As is apparent from the above test results, the new compounds (I) of the present invention are useful as anti-tumour agents.

For therapeutic administration, the new compounds (I) of the present invention and the pharmaceutically-acceptable salts thereof are used in a form of a conventional pharmaceutical preparation in admixture with a conventional pharmaceutically-acceptable carrier, such as an organic or inorganic solid or liquid excipient which is suitable for oral, parenteral or external administration.

The pharmaceutical preparation may be compounded in a solid form, such as a capsule, tablet, dragee or suppository, or in a liquid form, such as a solution, suspension or emulsion. If needed, the above preparations can also contain auxiliary substances, stabilising agents, wetting or emulsifying agents, buffers or any other commonly used additives.

The effective ingredient is usually administered in a unit dose of 1.0 mg/kg to 1000 mg/kg, 1 to 4 times a day. However, the above dosage may be increased or decreased according to the age, weight and condition of the patient or to the method of administration.

The following Examples are given for the purpose of illustrating the present invention:-

Example 1

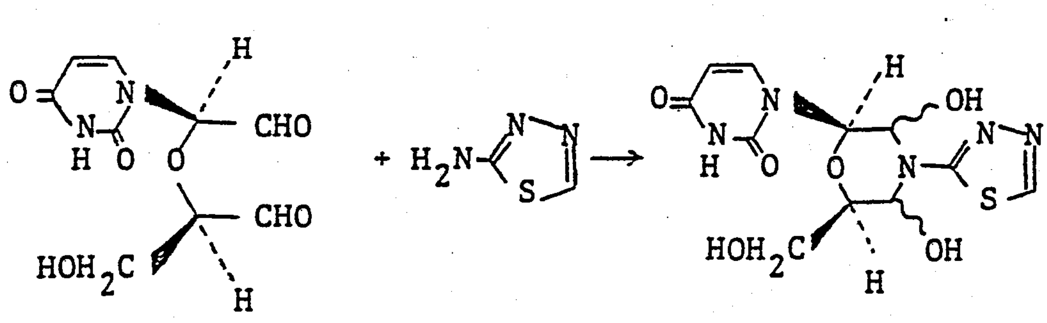

[R-(R*, R*)]-α-(1-Formyl-2-hydroxyethoxy)-3,4-dihydro-2,4-dioxo-1(2H)-pyrimidineacetaldehyde, (uridinedialdehyde), (2.42 g) was dissolved in a mixture of methanol (50 ml) and water (50 ml). To the solution was added 2-amino-1,3,4-thiadiazole (1.00 g). The mixture was stirred at ambient temperature for four hours and evaporated in vacuo. The residue was triturated with acetone to give 1-[(2R, 6R)-3,5-dihydroxy-6-hydroxymethyl-4-(1,3,4-thiadiazol-2-yl)morpholin-2-yl]-uracil (3.0 g). mp 170-180°C (dec.)

IR (Nujol) : 3200, 1690 $cm^{-1}$
NMR ($D_2O$) δ : 3.6-4.5 (3H, m), 5.0-5.7 (2H, m), 5.7-6.2 (2H, m), 7.1-8.1 (1H, m), 8.6-9.0 (1H, m)

Example 2

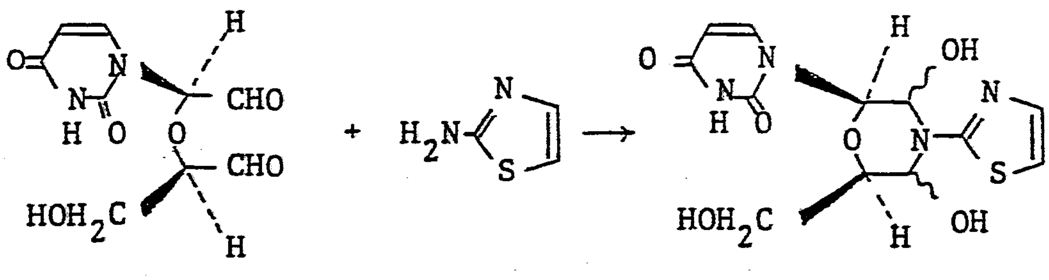

[R-(R*, R*)]-α-(1-Formyl-2-hydroxyethoxy)-3,4-dihydro-2,4-dioxo-1(2H)pyrimidineacetaldehyde, (uridinedialdehyde), (6.05 g) was dissolved in water (25 ml). To a solution was added 2-aminothiazole (2.50 g). The mixture was stirred at ambient temperature overnight to give a clear solution. The solution was subjected to column chromatography on HP-20 resine, which was eluted with water and then 50% aqueous acetone. The elution was condenced in vacuo and lyophilized to give white powder of 1-[(2R, 6R)-3,5-dihydroxy-6-hydroxymethyl-4-(2-thiazolyl)morpholin-2-yl]uracil (8.09 g). mp 130-135°C (dec.)

IR (Nujol) : 3250, 1690 $cm^{-1}$

NMR ($D_2O$)δ : 3.6-4.5 (3H, m), 5.1-5.8 (2H, m), 5.8-6.2 (2H, m), 6.7-7.5 (2H, m), 7.7-8.2 (1H, m)

Example 3

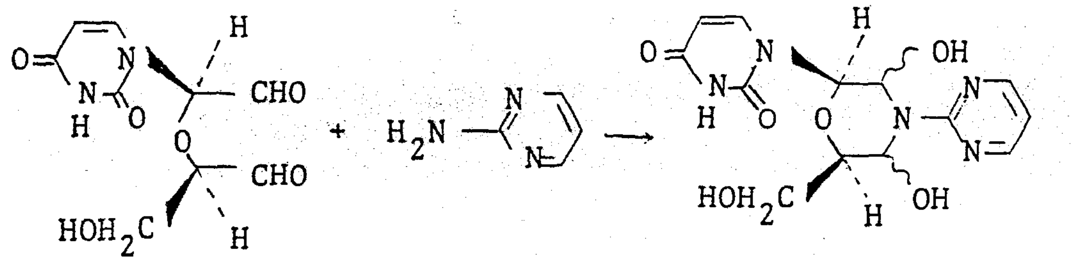

[R-(R*, R*)]-α-(1-Formyl-2-hydroxyethoxy)-3,4-dihydro-2,4-dioxo-1(2H)-pyrimidineacetaldehyde, (uridinedialdehyde), (6.05 g) was dissolved in water (25 ml). To the solution was added 2-aminopyrimidine (2.38 g). The mixture was stirred at ambient temperature for eight hours to give a clear solution. The solution was lyophilized to give white powder of 1-[(2R, 6R)-3,5-dihydroxy-6-hydroxymethyl-4-(2-pyrimidinyl)morpholin-2-yl]uracil (8.40 g).

mp 155-160°C (dec.)

IR (Nujol) : 3300, 1690, 1580 $cm^{-1}$

NMR ($D_2O$)δ : 3.7-4.4 (3H, m), 5.1-5.5 (1H, m), 5.5-6.4 (3H, m), 6.7-7.2 (1H, m), 7.7-8.2 (1H, m), 8.2-8.7 (2H, m)

Example 4

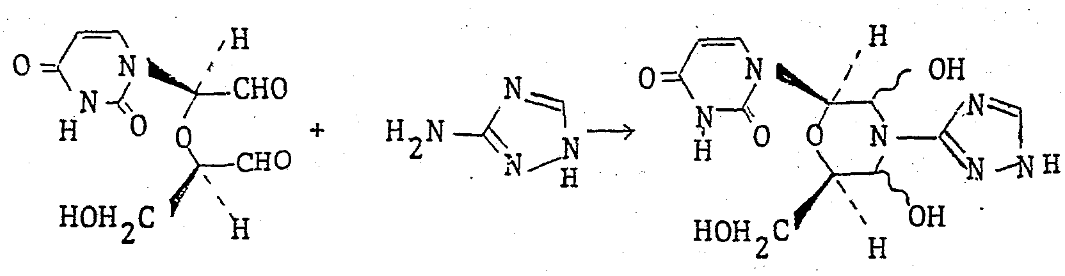

[R-(R*, R*)]-α-(1-Formyl-2-hydroxyethoxy)-3,4-dihydro-2,4-dioxo-1(2H)-pyrimidineacetaldehyde, (uridinedialdehyde), (4.84 g) was dissolved in a mixture

of methanol (100 ml) and water (100 ml). To the solution was added 3—amino-1H-1,2,4-triazole (1.68g). The mixture was stirred at ambient temperature for five hours and evaporated in vacuo. The residue was triturated with acetone to give 1-[(2R, 6R)-3,5-dihydroxy-6-hydroxymethyl-4-(1,2,4-triazol-3-yl)morpholin-2-yl]uracil (6.25 g). mp 210°C (dec.)

IR (Nujol) : 3200, 1680 $cm^{-1}$

NMR ($D_2O$)δ : 3.7-4.5 (3H, m), 5.0-6.5 (4H, m), 7.7-8.5 (2H, m)

Example 5

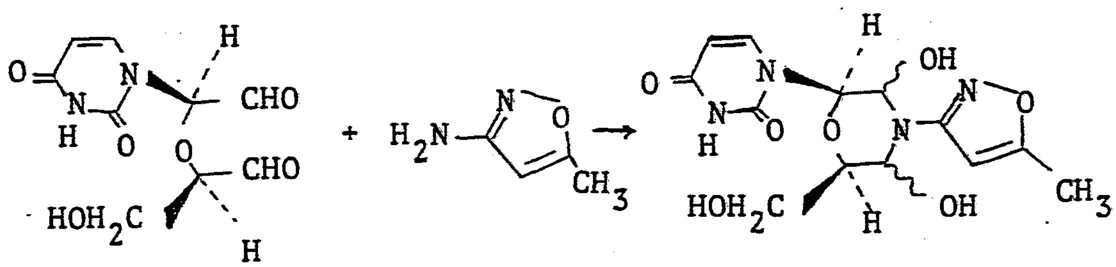

[R-(R*, R*)]-α-(1-Formyl-2-hydroxyethoxy)-3,4-dihydro-2,4-dioxo-1(2H)pyrimidineacetaldehyde, (uridinedialdehyde), (4.0 g) was dissolved in a mixture of methanol (30 ml) and water (30 ml). To the solution was added 3-amino-5-methylisoxazole (1.62 g). The mixture was stirred at ambient temperature for five hours and evaporated in vacuo. The residue was triturated with acetone to give 1-[(2R, 6R)-3,5-dihydroxy-6-hydroxymethyl-4-(5-methylisoxazol-3-yl)morpholin-2-yl]uracil (4.2 g). mp 163-167°C (dec.)

IR (Nujol) : 3300, 1690, 1620 $cm^{-1}$

NMR ($D_2O$)δ : 2.1-2.4 (3H, m), 3.6-4.5 (3H, m), 4.9-5.5 (2H, m), 5.5-6.2 (3H, m), 7.7-8.2 (1H, m)

Example 6

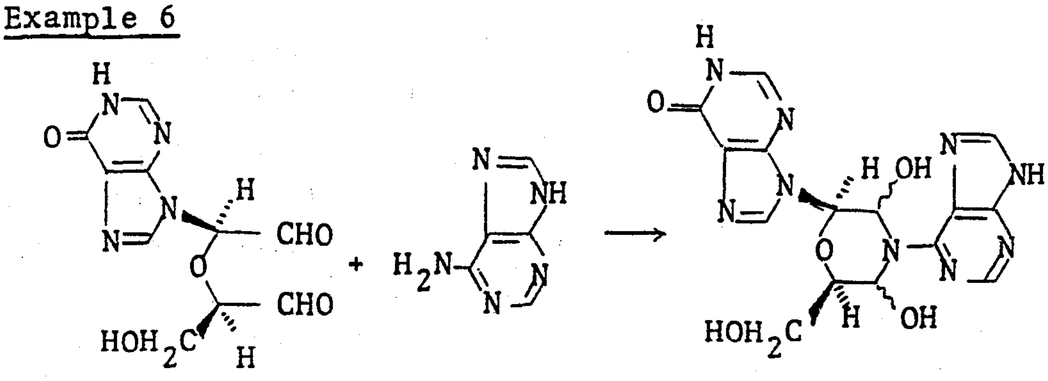

[R-(R*, R*)]-α-(1-Formyl-2-hydroxyethoxy)-1,6-dihydro-6-oxo-9H-purine-9-acetaldehyde, (inosinedialdehyde), (2.66 g) was dissolved in a mixture of methanol (100 ml) and water (100 ml). To the solution was added adenine (1.35 g). The mixture was stirred at ambient temperature for one day and filtered. The filtrate was evaporated in vacuo at 35°C. The residue was triturated with acetone to give 9-[(2R, 6R)-3,5-dihydroxy-6-hydroxymethyl-4-(6-purinyl)morpholin-2-yl]hypoxanthine(3.20 g). mp 125-135°C (dec.)

IR (Nujol) : 3150, 1690, 1590 $cm^{-1}$

NMR ($D_2O$)δ : 3.6-4.5 (3H, m), 5.0-6.5 (3H, m), 8.0-8.7 (4H, m)

Example 7

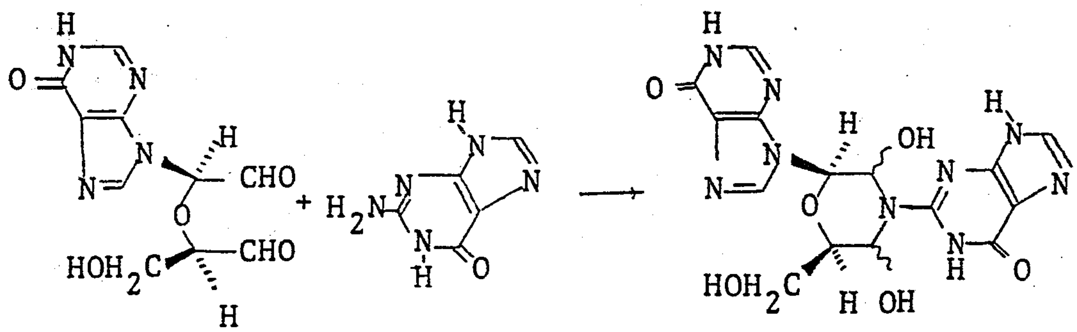

[R-(R*, R*)]-α-(1-Formyl-2-hydroxyethoxy)-1,6-dihydro-6-oxo-9H-purine-9-acetaldehyde, (inosinedialdehyde),

(1.33 g) was dissolved in a mixture of methanol (300 ml) and water (300 ml). To the solution was added guanine (0.76 g). The mixture was stirred at ambient temperature for three days and filtered. The filtrate was evaporated in vacuo at 35°C. The residue was triturated with acetone to give 9-[(2R, 6R)-3,5-dihydroxy-6-hydroxymethyl-4-(2-hypoxanthinyl)morpholin-2-yl]hypoxanthine (2.20 g). mp 115-120°C (dec.)

IR (Nujol) : 1310, 1680, 1580 $cm^{-1}$

NMR ($D_2O$)δ : 3.7-4.5 (3H, m), 5.0-6.3 (3H, m), 8.2-8.8 (3H, m)

Example 8

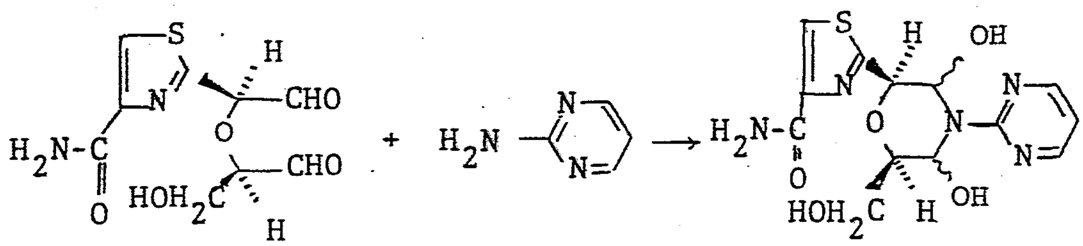

[R-(R*, R*)]-α-(1-Formyl-2-hydroxyethoxy)-4-carbamoyl-2-1,3-thiazoleacetaldehyde (1.10 g) was dissolved in water (30 ml). To the solution was added 2-aminopyrimidine (0.41 g). The mixture was stirred at ambient temperature for five hours to give a clear solution. The solution was lyophilized to give 2-[(2R,6R)-3,5-dihydroxy-6-hydroxymethyl-4-(2-pyrimidinyl)-morpholin-2-yl]-4-carbamoylthiazole (1.50 g). mp 105-110°C (dec.)

IR (Nujol) : 3320, 1680, 1630, 1580 $cm^{-1}$

NMR ($D_2O$)δ : 3.5-4.2 (3H, m), 4.5-6.0 (3H, m), 6.7-7.1 (1H, m), 8.1-8.8 (3H, m)

Example 9

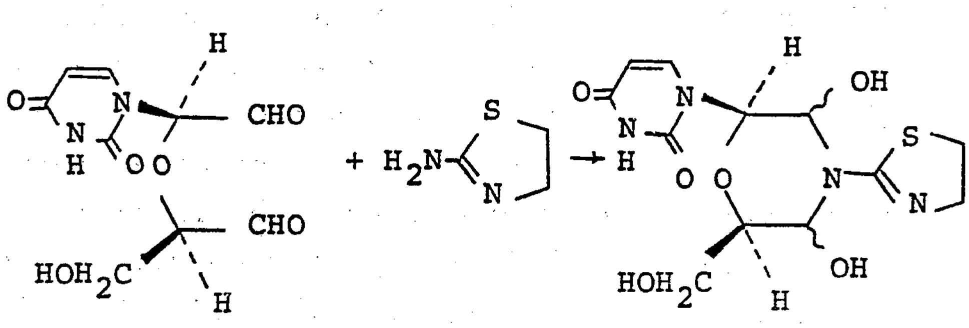

[R-(R*,R*)]-α-(1-Formyl-2-hydroxyethoxy)-3,4-dihydro-2,4-dioxo-1(2H)-pyrimidineacetaldehyde, (uridinedialdehyde), (2.4 g) was dissolved in water (10 ml). To the solution was added 2-amino-2-thiazoline (1.2 g). The mixture was stirred at ambient temperature overnight. An insoluble material was filtered off. The filtrate was evaporated in vacuo. The residue was triturated with acetone to give 1-[(2R,6R)-3,5-dihydroxy-6-hydroxymethyl-4-(2-thiazolin-2-yl)morpholin-2-yl]uracil (0.98 g).

IR (Nujol) : 3250, 1680 $cm^{-1}$

NMR ($DMSO-d_6-D_2O$) δ : 2.7-4.5 (7H, m), 4.5-6.0 (3H, m), 7.3-8.1 (2H, m)

Example 10

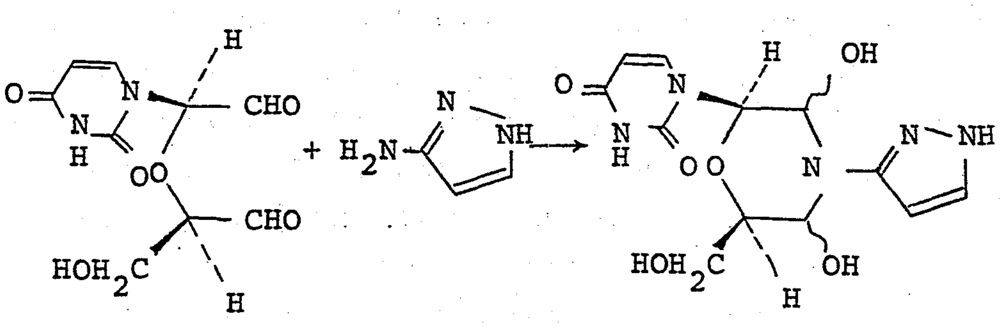

[R-(R*,R*)]-α-(1-Formyl-2-hydroxyethoxy)-3,4-dihydro-2,4-dioxo-1(2H)-pyrimidineacetaldehyde, (uridinedialdehyde), (2.4 g) was dissolved in water (10 ml). To the solution was added 3-aminopyrazole (0.83 g). The mixture was stirred at ambient temperature overnight. The precipitates were filtered and air-dried to give 1-[(2R,6R]-3,5-dihydroxy-6-hydroxymethyl-4-(3-pyrazolyl)morpholin-2-yl]uracil (1.25 g).

IR (Nujol) : 3250, 1680 $cm^{-1}$

NMR ($DMSO-d_6-D_2O$) δ : 2.7-4.6 (3H, m), 4.6-6.5 (3H, m), 7.0-8.0 (4H, m).

Example 11

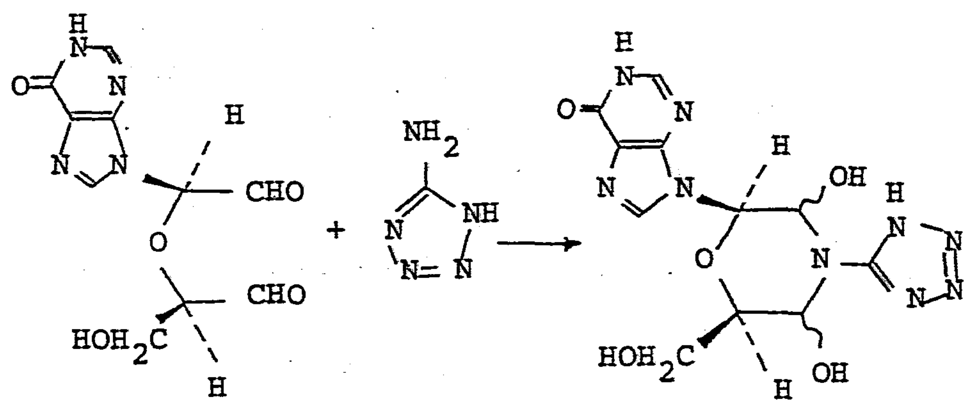

[R-(R*,R*)]-α-(1-Formyl-2-hydroxyethoxy)-1,6-dihydro-6-oxo-9H-purine-9-acetaldehyde, (inosinedialdehyde), (2.6 g) was dissolved in water (25 ml). To the solution was added 5-aminotetrazole (1.03 g) and stirred at ambient temperature overnight. The reaction mixture was subjected to column chromatography on macroporous non-ionic adsorption resin (Diaion HP-20, prepared by Mitsubishi Chem. Ind. Ltd.). The column was eluted with wat

The eluate was lyophilized to give 9-[(2R,6R)-3,5-dihydroxy-6-hydroxymethyl-4-(tetrazol-5-yl)-morpholin-2-yl]hypoxanthine (2.0 g).

IR (Nujol) : 3200, 1690 $cm^{-1}$

NMR ($D_2O$) δ : 3.6-4.7 (3H, m), 5.0-6.3 (3H, m), 8.0-8.8 (2H, m)

Example 12

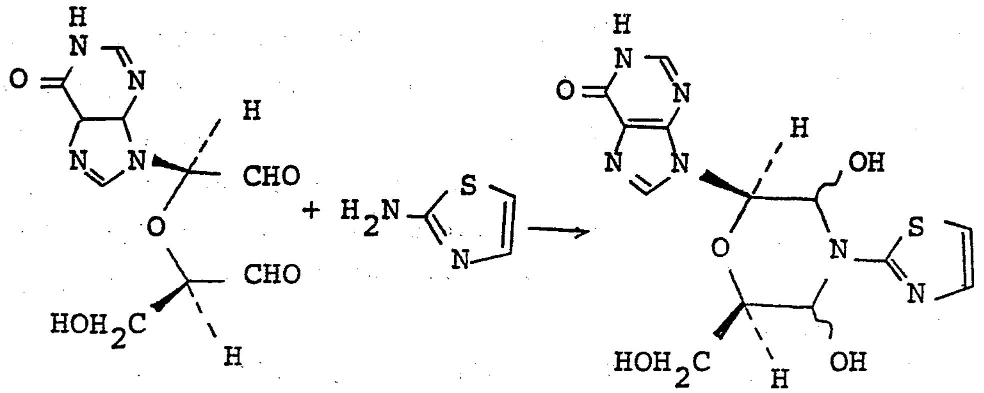

[R-(R*,R*)]-α-(1-Formyl-2-hydroxyethoxy)-1,6-dihydro-6-oxo-9H-purine-9-acetaldehyde, (inosinedialdehyde), (2.6 g) was dissolved in water (30 ml). To the solution was added 2-aminothiazole (1.0 g). The solution was adjusted at pH 5.0 with dilute hydrochloric acid and stirred at ambient temperature overnight. The precipitate was collected by filtration, washed with acetone and air-dried to give 9-[(2R,6R)-3,5-dihydroxy-6-hydroxymethyl-4-(2-thiazolyl)morpholin-2-yl]hypoxanthine (2.2 g).

IR (Nujol) : 3200, 1700 $cm^{-1}$

NMR (DMSO-$d_6$—$D_2O$) δ : 3.5-5.2 (3H, m), 5.2-6.5 (3H, m), 6.8-7.5 (2H, m), 8.0-8.3 (2H, m)

Example 13

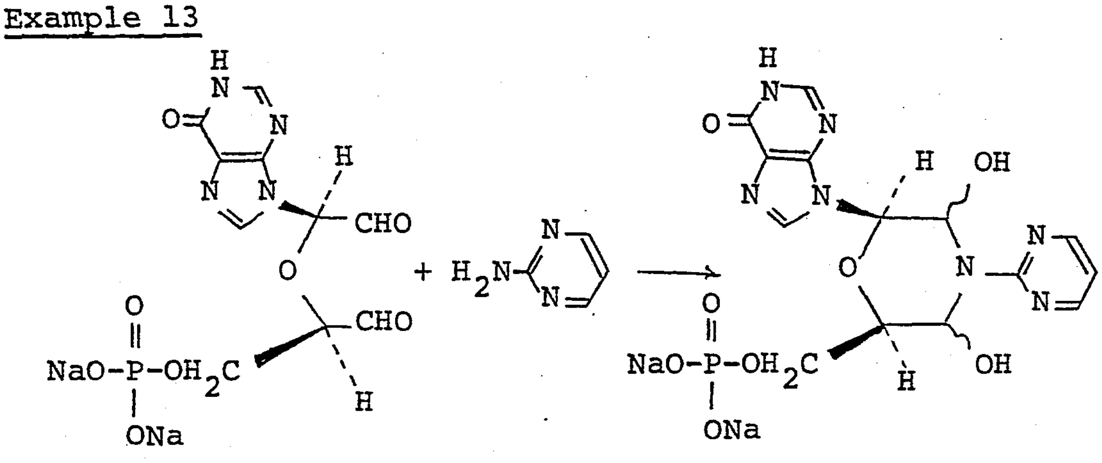

[R-(R*,R*)]-α-(1-Formyl-2-disodiophosphonoethoxy)-1,6-dihydro-6-oxo-9H-purine-9-acetaldehyde, (1.95 g) was dissolved in water (50 ml). To the solution was added 2-aminopyrimidine (0.48 g). The solution was stirred at ambient temperature for 1.3 hours and evaporated in vacuo. The residue was triturated with acetone and dried over phosphorus pentoxide in vacuo to give 9-[(2R,6R)-3,5-dihydroxy-6-disodiophosphonomethyl-4-(pyrimidin-2-yl)morpholin-2-yl]hypoxanthine (2.1 g).

IR (Nujol) : 3200, 1690, 1585 $cm^{-1}$

NMR ($D_2O$) δ : 3.5-4.7 (3H, m), 5.2-6.5 (3H, m), 6.6-7.1 (1H, m), 7.9-8.8 (4H, m)

Example 14

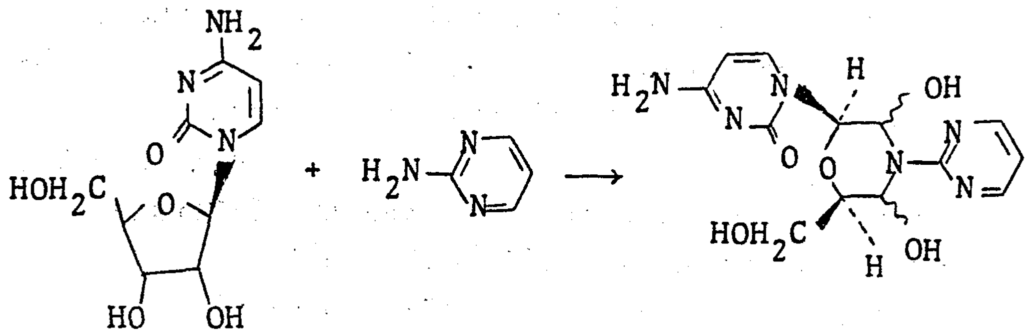

To a solution of cytidine (2.43 g) and 2-aminopyrimidine (0.95 g) in water (15 ml) was added sodium metaperiodate (2.13 g) at 15°C. The solution was stirred at ambient temperature for four hours. To the reaction mixture was added methanol (20 ml). A precipitate was filtered off and the filtrate was condenced in vacuo. The condenced solution was lyophilized to give white powder of 1-[(2R, 6R)-3,5-dihydroxy-6-hydroxymethyl-4-(2-pyrimidinyl)morpholin-2-yl]cytosine (3.20 g).

mp 145-150°C (dec.)

IR (Nujol) : 3250, 1640, 1580 $cm^{-1}$

NMR ($D_2O$) $\delta$: 3.7-4.3 (3H, m), 5.1-5.5 (1H, m), 5.5-6.4 (3H, m), 6.7-7.2 (1H, m), 7.7-8.2 (1H, m), 8.2-8.7 (2H, m)

Example 15

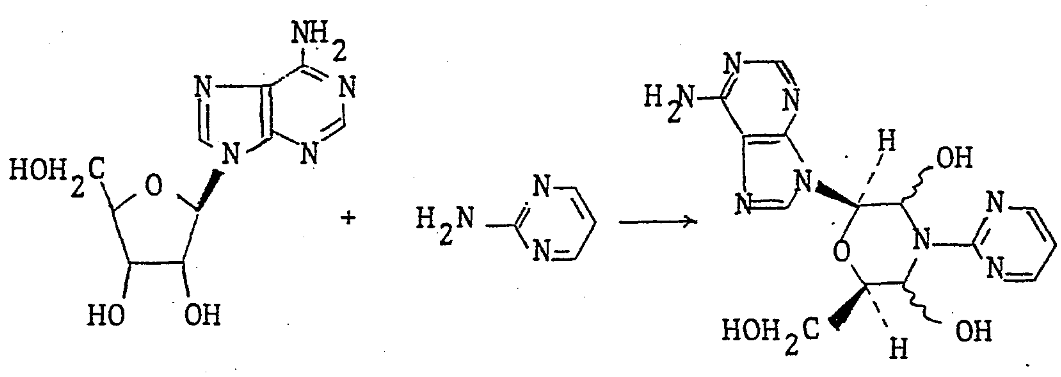

A solution of sodium metaperiodate (2.13 g) in water (25 ml) was added to a suspension of adenosine (2.67 g) and 2-aminopyrimidine in water (25 ml) at 15°C. The mixture was stirred at ambient temperature for five hours. To the reaction mixture was added methanol (50 ml). A precipitate was filtered off and the filtrate was evaporated in vacuo at 35°C. The residue was triturated with acetone to give 9-[(2R, 6R)-3,5-dihydroxy-6-hydroxymethyl-4-(2-pyrimidinyl)morpholin-2-yl]adenine (3.64 g). mp 155-160°C (dec.)

IR (Nujol) : 3350, 3200, 1640, 1580 $cm^{-1}$

NMR ($D_2O$)δ : 3.5-4.33 (3H, m), 5.0-6.2 (3H, m), 6.5-7.1 (1H, m), 8.0-8.8 (4H, m)

Example 16

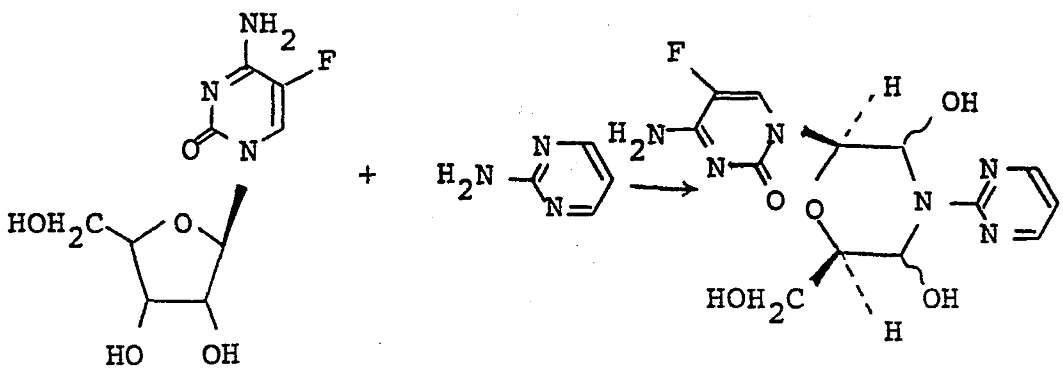

To a solution of 5-fluorocytidine (2.42 g) and 2-aminopyrimidine (0.95 g) in water (50 ml) was added sodium metaperiodate (2.13 g) under cooling with an ice-water bath. The solution was stirred at ambient temperature for 8 hours. To the reaction mixture was added methanol (50 ml). The precipitate was filtered off and the filtrate was evaporated in vacuo. The residue was triturated with acetone and air-dried to give 1-[(2R,6R)-3,5-dihydroxy-6-hydroxymethyl-4-(2-pyrimidinyl)morpholin-2-yl]-5-fluorocytosine (2.3 g).

IR (Nujol) : 3300, 1670, 1580 $cm^{-1}$

NMR ($D_2O$) δ : 3.6-4.5 (3H, m), 5.0-7.0 (4H, m), 7.5-7.7 (1H, m), 7.7-8.3 (2H, m)

## Patent Claims

1. Compounds of the formula:

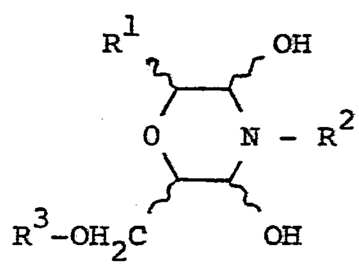

wherein $R^1$ is a uracilyl, cytosinyl, hypoxanthinyl, adeninyl or thiazolyl radical, which may be substituted by halogen or carbamoyl;
$R^2$ is a nitrogen-containing unsaturated heterocyclic radical, which may be substituted by oxo or lower alkyl; and
$R^3$ is a hydrogen atom or a phosphono group;

and the pharmaceutically-acceptable salts thereof.

2. Compounds according to claim 1, wherein $R^2$ is a nitrogen-containing unsaturated heterocyclic radical which is a 5- or 6-membered ring or is a fused ring comprising 5- or 6-membered rings, which may be substituted by oxo or lower alkyl.

3. A compound according to claim 2, wherein $R^2$ is a nitrogen-containing unsaturated heterocyclic radical selected from thiadiazolyl, thiazolyl, thiazolinyl, isoxazolyl, pyrazolyl, triazolyl, tetrazolyl, pyrimidinyl, purinyl and hypoxanthinyl.

4. A compound according to claim 3, wherein $R^2$ is thiadiazolyl, thiazolyl, tetrazolyl, pyrimidinyl, purinyl or hypoxanthinyl.

5. A compound according to claim 4, wherein $R^1$ is uracilyl, cytosinyl, hypoxanthinyl or thiazolyl, which may be substituted by halogen or carbamoyl.

6. A compound according to claim 5, wherein $R^1$ is uracilyl optionally substituted by halogen.

7. A compound according to claim 6, which is 1-[(2R,6R)-3,5-dihydroxy-6-hydroxymethyl-4-(2-pyrimidinyl)-morpholin-2-yl]-uracil or 1-[(2R,6R)-3,5-dihydroxy-6-hydroxymethyl-4-(1,2,4-triazol-3-yl)-morpholin-2-yl]-uracil or 1-[(2R,6R)-3,5-dihydroxy-6-hydroxymethyl-4-(1,3,4-thiadiazol-2-yl)-morpholin-2-yl]-uracil.

8. A compound according to claim 5, wherein $R^1$ is cytosinyl optionally substituted by halogen.

9. A compound according to claim 8, which is 1-[(2R,6R)-3,5-dihydroxy-6-hydroxymethyl-4-(2-pyrimidinyl)-morpholin-2-yl]-cytosine or 1-[(2R,6R)-3,5-dihydroxy-6-hydroxymethyl-4-(2-pyrimidinyl)-morpholin-2-yl]-5-fluorocytosine.

10. A compound according to claim 5, wherein $R^1$ is thiazolyl optionally substituted by carbamoyl.

11. A compound according to claim 10, which is 2-[(2R,6R)-3,5-dihydroxy-6-hydroxymethyl-4-(2-pyrimidinyl)-morpholin-2-yl]-4-carbamoylthiazole.

12. A compound according to claim 5, wherein $R^1$ is hypoxanthinyl.

13. A compound according to claim 12, which is 9-[(2R,6R)-3,5-dihydroxy-6-hydroxymethyl-4-(6-purinyl)-morpholin-2-yl]-hypoxanthine or 9-[(2R,6R)-3,5-dihydroxy-6-hydroxymethyl-4-(2-hypoxanthinyl)-morpholin-2-yl]-hypoxanthine.

14. A process for preparing compounds of the formula:

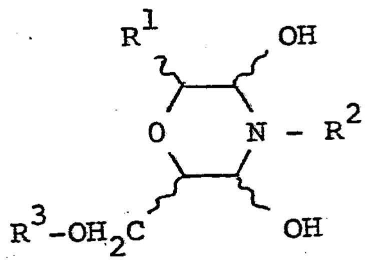

wherein $R^1$ is a uracilyl, cytosinyl, hypoxanthinyl, adeninyl or thiazolyl radical, which may be substituted by halogen or carbamoyl;
$R^2$ is a nitrogen-containing unsaturated heterocyclic radical, which may be substituted by oxo or lower alkyl; and
$R^3$ is a hydrogen atom or a phosphono group,
and pharmaceutically-acceptable salts thereof, which comprises

a) reacting a compound of the formula:

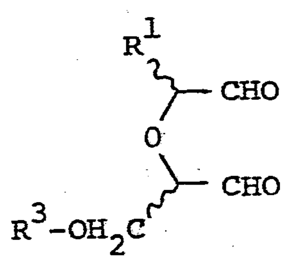

or a salt thereof with a compound of the formula:

$$H_2N-R^2$$

or a salt thereof to give a compound of the formula:

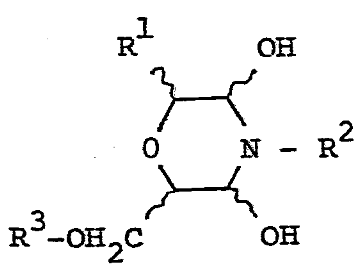

or a pharmaceutically acceptable salt thereof, $R^1$, $R^2$ and $R^3$ in the above formulae being as defined above; or

b) reacting a compound of the formula:

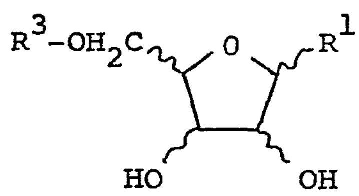

or a salt thereof with a compound of the formula:

$$H_2N-R^2$$

or a salt thereof in the presence of an oxidising agent to give a compound of the formula:

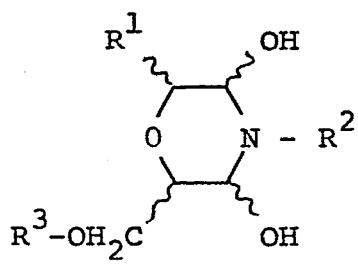

or a pharmaceutically-acceptable salt thereof, $R^1$, $R^2$ and $R^3$ in the above formulae being as defined above.

15. A pharmaceutical composition comprising a compound according to claim 1 as the effective ingredient, in association with a pharmaceutically-acceptable, substantially non-toxic carrier or excipient.

16. A method for the treatment of cancer, which comprises administering an effective amount of a compound according to claim 1 to a human being or to an animal.